(19) Europäisches Patentamt
European Patent Office
Office européen des brevets

(11) EP 1 536 791 B1

(12) **EUROPEAN PATENT SPECIFICATION**

(45) Date of publication and mention of the grant of the patent:
**27.01.2010 Bulletin 2010/04**

(21) Application number: **03771898.8**

(22) Date of filing: **25.07.2003**

(51) Int Cl.:
***A61K 31/428*** (2006.01) ***A61K 9/20*** (2006.01)
***A61K 9/28*** (2006.01) ***A61K 31/5375*** (2006.01)
***A61K 31/4745*** (2006.01)

(86) International application number:
**PCT/US2003/023418**

(87) International publication number:
**WO 2004/010998 (05.02.2004 Gazette 2004/06)**

(54) **SUSTAINED-RELEASE TABLET COMPRISING REBOXETINE**

REBOXETIN ENTHALTENDE TABLETTE MIT VERZÖGERTER WIRKSTOFFABGABE

COMPRIME A LIBERATION PROLONGEE COMPRENANT DE LA REBOXETINE

(84) Designated Contracting States:
**AT BE BG CH CY CZ DE DK EE ES FI FR GB GR HU IE IT LI LU MC NL PT RO SE SI SK TR**

(30) Priority:
**25.07.2002 US 398427 P**
**25.07.2002 US 398447 P**
**28.08.2002 US 406609 P**
**18.06.2003 US 479387 P**

(43) Date of publication of application:
**08.06.2005 Bulletin 2005/23**

(60) Divisional application:
**09178277.1**

(73) Proprietor: **Pharmacia Corporation**
**Peapack, NJ 07977 (US)**

(72) Inventors:
- **AMIDON, Gregory, E.**
**Portage, MI 49024 (US)**
- **GANORKAR, Loksidh, D.**
**Kalamazoo, MI 49009 (US)**
- **HEIMLICH, John, M.**
**Portage, MI 49002 (US)**
- **LEE, Ernest, J.**
**Kalamazoo, MI 49009 (US)**
- **MARTINO, Alice, C.**
**Kalamazoo, MI 49009 (US)**
- **NOACK, Robert, M.**
**Grand Rapids, MI 49506 (US)**
- **REO, Joseph, P.**
**Kalamazoo, MI 49009 (US)**
- **SKOUG, Connie, J.**
**Portage, MI 49024 (US)**

(74) Representative: **Summers, Victoria Clare**
**Pfizer Limited**
**European Patent Department**
**Ramsgate Road**
**Sandwich, Kent CT13 9NJ (GB)**

(56) References cited:
**EP-A- 0 933 079** **WO-A-00/59477**
**WO-A-01/01973** **WO-A-01/19337**
**WO-A-01/22820** **WO-A-90/15058**
**WO-A-97/04752** **WO-A-99/09066**
**WO-A-99/16442** **WO-A-99/45924**
**US-A- 2 887 440** **US-A- 4 731 374**
**US-A- 5 472 712** **US-B1- 6 277 875**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 536 791 B1

## Description

### FIELD OF THE INVENTION

The present invention relates to tablet formulations, and more particularly to a sustained-release tablet composition for oral delivery of a water-soluble drug or prodrug.

### BACKGROUND OF THE INVENTION

Many active pharmaceutical agents, including drugs and prodrugs, have been formulated as orally deliverable dosage forms providing sustained release (otherwise known as slow release or extended release) of such agents over a period of time effective to permit once daily administration. A well-known system for formulating such dosage forms involves a matrix comprising a hydrophilic polymer wherein the agent is dispersed; the agent is released over a period of time in the gastrointestinal tract upon dissolution or erosion of the matrix. Sustained-release dosage forms comprising such a matrix system are conveniently prepared as compressed tablets, described herein as "matrix tablets".

Drugs and prodrugs having relatively high solubility in water, for example a solubility of about 10 mg/ml or greater, present challenges to the formulator wishing to provide a sustained-release dosage form, and the higher the solubility the greater are the challenges. These challenges are well illustrated in the cases of pramipexole dihydrochloride, which has a solubility in water of about 200 mg/ml, and reboxetine mesylate, which has a solubility in water of about 250 mg/ml.

Pramipexole (I) is a dopamine $D_2$ receptor agonist useful in treatment of Parkinson's disease. Pramipexole as its dihydrochloride salt is commercially available in the United States as Mirapex® tablets of Pharmacia & Upjohn. These are immediate-release tablets in 0.125 mg, 0.25 mg, 0.5 mg, 1.0 mg and 1.5 mg strengths, designed for oral administration of a single tablet three times per day to provide a daily dose of 0.375 to 4.5 mg. See Physicians' Desk Reference 57th edition (2003), 2768-2772. Doses herein are expressed in amounts ofpramipexole dihydrochloride monohydrate unless otherwise specified; 1.0 mg pramipexole dihydrochloride monohydrate is equivalent to about 0.7 mg pramipexole base.

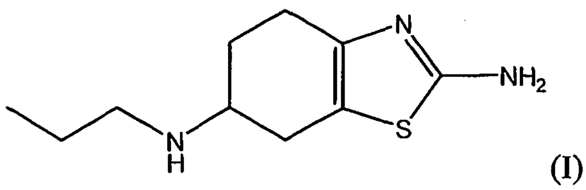

(I)

A three times daily dosing regimen for immediate-release pramipexole dihydrochloride tablets is well tolerated, but patient compliance would be much improved if a once-daily regimen were possible. In this regard, it will be noted that the primary indication for the drug, Parkinson's disease, is an affliction that becomes more prevalent with advancing age and is often accompanied by decline in memory. A once-daily regimen would be especially useful in enhancing compliance among elderly patients.

It has been found by the present inventors that formulation of pramipexole dihydrochloride monohydrate in a hydrophilic matrix tablet is generally inadequate to provide sustained-release properties consistent with once-daily dosing. Release characteristics can be further modified by coating the tablet with a sustained-release coating. Such a coating typically comprises a hydrophobic polymer and a hydrophilic pore-former.

The need to provide a coating over the matrix tablet gives rise to further problems. The additional handling operations involved in a coating step require a sufficient degree of tablet hardness to avoid tablet breakage and/or attrition during these operations, particularly in a high-speed manufacturing situation.

Reboxetine (II) is a selective noradrenaline reuptake inhibitor (SNRI) useful in treatment of depressive illness. Reboxetine as its methanesulfonate (mesylate) salt is commercially available in the United Kingdom and elsewhere as Edronax® tablets of Pharmacia & Upjohn. These are immediate-release tablets having a breaking score to facilitate division. Each Edronax® tablet contains 4 mg reboxetine and is designed for twice daily oral administration to provide a daily dose of 4 to 12 mg, with division of tablets if necessary. See British National Formulary 41st edition (2001), 196. Doses herein are expressed in amounts of reboxetine base unless otherwise specified.

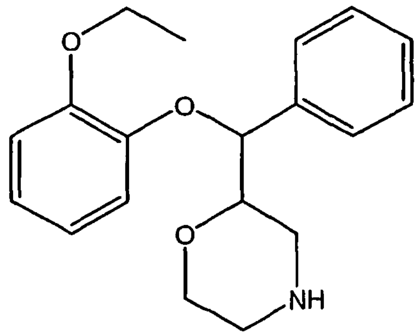

(II)

A twice-daily dosing regimen for immediate-release reboxetine tablets is well tolerated, but patient compliance would be much improved if a once-daily regimen were possible without substantially increasing the potential for adverse side effects. In this regard, it will be noted that the primary indication for the drug, depressive illness, is an affliction that is often accompanied by poor compliance.

It has been found by the present inventors that formulation of reboxetine salts in a hydrophilic matrix tablet can provide sustained-release properties consistent with once-daily dosing. However, the resulting tablets are susceptible to breakage and/or attrition during handling, especially in a high-speed tableting operation.

It has proved difficult to formulate a tablet having a suitable combination of sustained-release and handling properties, where the drug is one having relatively high solubility, as in the case of salts of pramipexole or reboxetine.

U.S. Patent No. 6,197,339 discloses a sustained-release tablet comprising (R)-5,6-dihydro-5-(methylamino)-4H-imidazo[4,5-ij]-quinolin-2(1H)-one (Z)-2-butenedioate (1:1) (sumanirole maleate) in a matrix comprising hydroxypropylmethylcellulose (HPMC) and starch. The tablet is disclosed to be useful in treatment of Parkinson's disease. Stanches disclosed to be suitable therein include pregelatinized starch.

U.S. Patent No. 5,458,887 discloses a controlled-release tablet comprising an osmotic core that consists of a drug in admixture with a water-swellable component such as HPMC or polyethylene oxide, and a coating that comprises a water-resistant polymer and a manor amount of a water-soluble compound that acts as a pore-former. Upon formation of pores in the coating by dissolution of the water-soluble compound, the water-swellable agent is said to expand the core and provide a drug-rich surface in contact with gastrointestinal fluid.

U:S. Patent No. 5,656,296 discloses a dual control sustained-release formulation comprising a core that comprises a drug and a low melting point excipient, and a coating layer over the core that comprises a pH-independent water-insoluble polymer and a water-soluble film-forming polymer.

European Patent Application No, EP 0 933 079 discloses a starch said to be suitable for preparing tablets having high hardness yet being capable of rapid disintegration in an aqueous medium, Tensile strength of the finished tablets is calculated from the hardness.

It is an object of the present invention to provide a sustained-release tablet composition of a water-soluble drug or prodrug that is suitable for once-daily oral administration. It is a further object to provide such a composition having sufficient hardness to withstand a high-speed tableting operation, in particular to resist erosion during application of a coating layer. It is a further object to provide a pharmaceutical tablet that provides day-long therapeutic effect on the central nervous system (CNS) of a subject when administered once daily. It is particular object to provide such a tablet that provides day-long therapeutic effect as a dopamine agonist when administered once daily, most particularly where the water-soluble drug is a salt of pramipexole, without substantially increased incidence of adverse side effects. It is another particular object to provide such a tablet that provides therapeutic effect as an SNRI when administered once daily, most particularly where the water-soluble drug is a salt of reboxetime or an enantiomer thereof, for example (S, S)-reboxetine, without substantially increased incidence of adverse side effects. It is a still further object to provide a method for testing a starch to assess its suitability for inclusion in a matrix tablet for sustained release of a water-soluble drug or prodrug.

SUMMARY OF THE INVENTION

There is now provided a sustained-release pharmaceutical composition in a form of an orally deliverable tablet for treating a CNS condition or disorder comprising an active pharmaceutical agent which is reboxetine or a salt thereof, the active pharmaceutical ingredient being dispersed in a matrix comprising a hydrophilic polymer and a starch having a tensile strength of at least about 0.15 kN $cm^{-2}$ at a solid fraction of 0.75 to 0.85 of the tablet. The composition preferably exhibits sustained-release properties adequate to provide therapeutic effectiveness when administered orally not more than once daily to a subject in need thereof.

There is further provided a process for preparing such sustained-release pharmaceutical compositions in a form of an orally deliverable tablet, the process comprising selecting by a suitable test a starch having a tensile strength of at least about 0.15 kN $cm^{-2}$ at a solid fraction of 0.75 to 0.85 of the tablet admixing the selected starch a hydrophilic polymer and an active pharmaceutical agent which is reboxetine or a salt thereof to provide a mixture wherein the agent is dispersed in a matrix comprising the polymer and the starch; and compressing the mixture to form a tablet.

A particularly convenient test method, comprises preparing compacts of a starch sample on an automated tablet press at a range of compression forces, measuring hardness of the compacts, determining solid fraction of the compacts, calculating tensile strength of the compacts from hardness and dimensions of the compacts, determining relationship of tensile strength to solid fraction of the compacts, and from that relationship estimating tensile strength at a solid fraction representative of a desired tablet.

An "active pharmaceutical agent" herein can be a drug or a prodrug or a salt thereof, including diagnostic agents. Unless otherwise specified, "solubility" herein means solubility in water at 20-25°C at any physiologically acceptable pH, for example at any pH in the range of 4 to 8. In the case of an agent that is a salt, reference herein to solubility in water pertains to the salt, not to the free acid or base form ofthe agent.

The term "orally deliverable" herein means suitable for oral, including peroral and intra-oral (e.g., sublingual or buccal) administration, but tablets of the present invention are adapted primarily for peroral administration, *i.e.*, for swallowing, typically whole or broken, with the aid of water or other drinkable fluid,

A "compact" herein is a compressed tablet, prepared for example on a tablet press, consisting only of a sample of starch for which it is desired to measure tensile strength. "Solid fraction" is the ratio of absolute to apparent density of a compact. A "solid fraction representative of the tablet" is a solid fraction selected to be similar to the solid fraction of tablets prepared according to the invention. Typically a solid fraction of 0.75 to 0.85, illustratively 0.8, will be selected.

A "subject" herein is an animal of any species, preferably mammalian, most preferably human Conditions and disorders in a subject for which a particular agent is said herein to be "indicated" are not restricted to conditions and disorders for which the agent has been expressly approved by a regulatory authority, but also include other conditions and disorders known or believed by a physician to be amenable to treatment with the agent. "Treatment" herein embraces prophylactic treatment unless the context requires otherwise.

## BRIEF DESCRIPTION OF THE DRAWINGS

Fig. 1 is a graph showing relationship of tensile strength of pregelatinized starch lots, as determined by a test method of the invention using a 4 second dwell time (Example 1 herein) to triaxial tensile strength.

Fig. 2 is a graph showing relationship of tensile strength of pregelatinized starch lots, as determined by a test method of the invention using a 90 second dwell time (Example 1 herein) to triaxial tensile strength,

Fig. 3 is a graph showing correlation of tensile strength of pregelatinized starch lots with maximum hardness of tablets containing these lots.

Fig. 4 is a graph showing *in vitro* dissolution profiles of three different 0.375 mg sustained-release tablet formulations of pramipexole dihydrochloride monohydrate, as more fully described in Example 10.

Fig. 5 is a graph showing *in vitro* dissolution profiles of three different 4 mg sustained-release tablet formulations of (S,S)-reboxetine in the form of its succinate salt, as more fully described in Example 12.

## DETAILED DESCRIPTION OF THE INVENTION

The invention provides a pharmaceutical composition in a form of an orally deliverable tablet comprising a water-soluble active pharmaceutical agent. The composition preferably exhibits sustained-release properties adequate to provide therapeutic effectiveness when administered orally not more than once daily.

Typically, agents for which the invention is especially useful are unsuitable for once daily administration when formulated in an immediate-release composition. This unsuitability can arise from one or more properties of such agents including without limitation:

(a) short half-life ($T_{1/2}$) of the agent or active metabolite thereof in the bloodstream, requiring the plasma concentration to be "topped up" at intervals shorter than one day to maintain a therapeutically effective concentration; and
(b) potential for undesirable side effects arising from a high maximum plasma concentration ($C_{max}$) of the agent or therapeutically active metabolite thereof

Relatively few agents having solubility not less than 10 mg/ml are non-ionizable compoumds. Most are compounds that exist as free acid or free base form and are present in such form, or more commonly in the form of a pharmaceutically acceptable salt, in a composition of the invention. Solubility of preferred agents is not less than 50 mg/ml, more preferably not less than 100 mg/ml. For purposes of the present invention, agents classified in the United

States Pharmacopeia, 24th edition (2000) (USP 24) as "very soluble" or "freely soluble" are considered to have solubility not less than 100 mg/ml, and together with agents classified in USP 24 as "soluble" or "sparingly soluble" are considered to have solubility not less than 10 mg/ml.

Active pharmaceutical agents useful herein are reboxetine or a salt thereof.

It will be understood that mention of an active pharmaceutical agent herein embraces racemates, enantiomers, polymorphs, hydrates and solvates thereof.

The present invention is especially suitable for highly potent drugs and prodrugs, *i.e.*, those that are therapeutically effective in low daily dosage amounts, for example not greater than 100 mg/day, especially not greater than 50 mg/day, more especially not greater than 25 mg/day, even more especially not greater than 10 mg/day, and most especially not greater than 5 mg/day.

In one embodiment the active pharmaceutical agent has therapeutic effect on the central nervous system (CNS). Such agents, herein referred to as "CNS agents", are useful in treatment or prevention of CNS disorders, and include without limitation anticonvulsant, antidepressant, antidyskinetic, antiepileptic, antimanic, antimigraine, antimuscarinic, antiobsessional, antiparkinsonian, antipsychotic, antispasmodic, anxiolytic, cholinergic, CNS stimulant, dopamine receptor agonist, dopamine receptor antagonist, hypnotic, monoamine oxidase inhibitor, neuroleptic, neuroprotective, NMDA receptor antagonist, nootropic, prolactin inhibitor, sedative, selective serotanin reuptake inhibitor (SSRI), selective noradrenaline reuptake inhibitor (SNRI), serenic, serotonin receptor agonist, serotonin receptor antagonist and tranquilizer agents.

Illustrative CNS agents useful herein include salts of reboxetine.

Reboxetine (II) can be used in the form of a racemic mixture comprising two or more of (R,R)-reboxetine, (S, S)-reboxetine, (R,S)-reboxetine and (S,R)-reboxetine, or in the form of any of these enantiomers alone. Preferably (S, S)-reboxetine is used.

Suitable salts of reboxetine include hydrochloride, hydrobromide, hydroiodide, sulfate, phosphate, acetate, propionate, lactate, maleate, malate, succinate, fumarate, tartrate, cyclohexanesulfamate, mesylate, esylate, besylate and tosylate salts. In the case of a reboxetine racemate, the mesylate salt is preferred. In the case of (S,S)-reboxetine, the succinate and fumarate salts, more especially the succinate salt, are preferred.

Reboxetine and (S,S)-reboxetine compositions af the invention are preferably suitable for administration no more than once daily. Such compositions are useful in treatment of any CNS condition or disorder for which reboxetine and enantiomers thereof have therapeutic utility, but especially depressive illness and neuropathic pain, including postherpetic neuralgia and diabetic neuropathy.

All active pharmaceutical agents useful herein can be prepared by processes known *per se*, including processes disclosed in patents and other literature pertaining to specific agents of interest.

The amount of the active pharmaceutical agent present in a composition ofthe invention depends on the potency of the agent, but is sufficient to provide a daily dose in one to a small plurality, for example one to 4, of tablets to be administered at one time. Preferably the full daily dose is delivered in a single tablet, In most cases the amount of the agent per tablet is 0.1 to 200 mg, preferably 0.2 to 100 mg. Expressed as percentage by weight of the composition, the amount of the agent is typically 0.01% to 25%, preferably 0.05% to 20%. In the case of an agent that is a salt, amounts of agent herein are expressed as free acid or free base equivalent amounts, unless otherwise specified.

In the case of reboxetine or (S,S)-reboxetine, an amount of 0.2 to 15 mg per tablet, or 0.1% to 10% by weight ofthe composition, will generally be suitable. Preferably an amount of 1 to 12 mg reboxetine or (S,S)-reboxetine per tablet is present. Specific dosage amounts per tablet contemplated herein include 1, 2, 4, 6, 8 and 12 mg reboxetine in the form of its mesylate salt or (S, S)-reboxetine in the form of its succinate salt.

A composition of the present invention comprises an active pharmaceutical agent as defined above, dispersed in a matrix comprising a hydrophilic polymer and a starch having a tensile strength of at least 0.15 kN $cm^{-2}$ at a solid fraction representative of the tablet, for example 0.75 to 0.85, illustratively 0.8.

Hydrophilic polymers useful herein are pharmaceutically acceptable polymeric materials having a sufficient number and distribution of hydrophilic substituents such as hydroxy and carboxy groups to impart hydrophilic properties to the polymer as a whole. Suitable hydrophilic polymers include, without limitation, methylcellulose, HPMC (hypromellose), carmellose (carboxymethylcellulose) sodium and carbomer (polyacrylic acid). More than one such polymer can optionally be used.

HPMC is a preferred hydrophilic polymer. Various types and grades of HPMC are available. In one embodiment HPMC type 2208, preferably meeting specifications set forth in a standard pharmacopeia such as USP 24, is used. HPMC type 2208 contains 19-24% by weight methoxy and 4-12% by weight hydroxypropoxy substituents. Especially suitable HPMC have nominal viscosity ranging from about 100 to about 10,000 mPa s; illustratively a suitable HPMC type 2208 is one having a nominal viscosity of about 4,000, with a measured viscosity of about 3,000 to about 5,600 mPa s. Such an HPMC is available, for example, as Methocel® K4MP from Dow Chemical Co., and substantially equivalent products are available from other manufacturers.

The amount of hydrophilic polymer in the composition depends on the particular polymer selected, on the active

pharmaceutical agent and on the desired sustained release profile. Typically, however, the hydrophilic polymer is included in an amount of 20% to 70%, preferably 30% to 60% and more preferably 35% to 50%, by weight of the composition. In the illustrative case of HPMC type 2208, a suitable amount will generally be found in the range from 30% to 60%, preferably 35% to 50%, for example 40%, by weight of the composition.

It is believed, without being bound by theory, that the hydrophilic polymer functions to provide extended or sustained release of the active pharmaceutical agent, for example by gradual dissolution or erosion of the polymer in the gastrointestinal tract.

Starches useful herein include starches from any suitable botanical source, for example corn, wheat, nice, tapioca, potato, *etc.*. Preferred starches have a relatively high ratio of amylose to amylopectin, containing for example at least 20%, more preferably at least 25%, amylose. Especially preferred is pregelatinized starch, which is a type of modified starch that has been processed to render the starch more flowable and directly compressible. Partially or wholly pregelatinized starches can be used.

It is believed, without being bound by theory, that the primary function of the starch in a composition of the invention is as a binding agent. A starch meeting the tensile strength criterion defined herein can be referred to as a "super binder".

The amount of starch in the composition is typically higher than is conventionally present as a binder in tablet formulations. Suitable amounts will generally be found in the range of 25% to 75% by weight. Preferably the amount of starch is 40% to 70%, more preferably 45% to 65%, for example 50%, by weight of the composition.

Tensile strength of a starch sample can be measured by any suitable test. Illustrative test procedures are described by Hiestand & Smith (1984), Powder Technology 38, 145-159, and by Hiestand & Smith (1991), International Journal of Pharmaceutics 67, 231-246, these articles being incorporated herein by reference.

An example of a tensile strength test that can be used (herein referred to as a "triaxial tensile strength test") requires preparation of a series of compacts of the starch sample, followed by determination of tensile strength ofthe compacts using a computerized multifunction tablet tester (MTT). The compacts are prepared with various degrees of compression force to provide compacts having a range of solid fraction. As a sustained release tablet formulation typically has a solid fraction of 0.8, it is useful to prepare compacts approximating such a solid fraction.

Absolute density of the starch sample can be determined using a helium-air pycnometer.

A computer-controlled triaxial tablet press is used to prepare the compacts. Voltage output from the punch and die load cells of the tablet press are first zeroed. The punch and die are lubricated with magnesium stearate powder and the die assembly is placed in the press. Compression and decompression parameters are selected on the computer. The desired amount of starch to be compacted is weighed and poured into the die cavity. The resulting powder bed is leveled with a spatula. The punch is inserted into the die and the computer-controlled compression/decompression cycle is started,

Just prior to the end of the compression phase, thickness of the compact as measured by LVDT is recorded. At the end of the compression phase, the final compression force as measured by voltage of the punch load cell is recorder.

At the end of the decompression phase, the punch and die rams are retracted. The compact is removed from the die and inspected for defects, such as cracking or sticking. Cracking can be reduced by increasing decompression time. If the compact is free of defects, its length, width, thickness and weight are measured to enable calculation of apparent density, Solid fraction is calculated by dividing absolute density by apparent density.

In preparation of the MTT for tensile strength determination, a suitable software program is run. The platen is screwed to the load cell of the MTT and the tensile strength assembly is slid into the MTT opposite the platen. The load cell signal is monitored via the computer and the zero offset on the signal conditioner is adjusted to provide a positive baseline voltage as close as possible to zero. A forward velocity is selected that will generate a time constant of approximately 15 seconds (usually the velocity selected will be 0.8 to 1.2 mm $s^{-1}$).

The compact to be tested is placed in the holder of the tensile strength assembly. The motor is initiated via the computer, driving the platen toward the compact until the surface of the compact is detected, and stopping the platen a few millimeter from the compact. The oscilloscope is triggered, to record the force applied to the compact, and the motor is restarted. The platen is driven into the compact until a crack is detected, either by sight or by sound, and the motor is immediately reversed.

Peak force is recorded from the oscilloscope trace. Tensile strength is calculated from the peak force using appropriate computer software.

From several runs using compacts at a range of solid fractions around 0.8, data are plotted and tensile strength at a solid fraction of 0.8 is estimated. If the tensile strength at a solid fraction of 0.8 is 0.15 kN $cm^{-2}$ or greater, the starch sample is deemed to be suitable for use in preparing a composition according to the invention.

It has now surprisingly been discovered that a much simpler test, one that is more amenable to implementation in a manufacturing setting, can be used to estimate tensile strength of a starch sample, in particular to determine whether the starch sample has a tensile strength of at least 0.15 kN $cm^{-2}$ at a solid fraction representative of a desired sustained-release tablet.

According to this test, compacts ofthe starch sample are prepared on a standard automated tablet press under a range of compression forces. For example, a Carver press (*e.g.*, Model 3888.1DT0000) fitted with flat-meed tooling of suitable diameter (*e.g.*,10/32 inch or about 0.7 cm for a 300 mg compact), operated at compression forces of 4 to 16 kN (900 to 3600 lbf) for a dwell time of at least about 4 seconds has been found to give satisfactory result. Illustratively, such compacts can be prepared at 1000, 1500, 2000 and 3000 lbf (4.45, 6.67, 8.90 and 13.34 kN). Preferably a dwell time of at least 10 seconds, more preferably at least 30 seconds, still more preferably at least 60 seconds, is used. Illustratively, a dwell time of 90 seconds has been found to give satisfactory results. Weight, diameter and thickness of each compact are measured accurately (alternatively, diameter can be assumed to equal that of the tooling) to enable calculation of apparent density and hence solid fraction, absolute density having been measured as described above, for example by helium-air pycnometry.

Hardness of each compact thus prepared is then determined by any suitable tablet hardness test, for example using a Key HT 500 hardness tester. Hardness is a measure of the force required to cause crushing of the compact, and is typically expressed in units such as kiloponds (kp) or Strong-Cobb units (SCU). A hardness of about 10.2 kp or about 14.4 SCU corresponds to a force of 0.1 kN.

For present purposes it is considered that crushing strength of the compact is equivalent to tensile strength. Thus tensile strength ($\sigma_T$, in kN $cm^{-2}$) can be calculated from the equation

$$\sigma_T = 2F/\pi DH$$

where F is the force required to cause crushing (in kN), D is diameter of the compact (in cm) and H is thickness ofthe compacts (in cm). For example, a compact of diameter 0.7 cm and thickness 0.4 cm having a hardness of 20 SCU (equivalent to a force of 0.139 kN) has a calculated tensile strength of 0.316 kN $cm^{-2}$.

The relationship between tensile strength and solid fraction is next established for the starch sample. This can be done by plotting data for tensile strength and solid fraction on a graph (solid fraction tends to increase with increasing compression force during preparation of the compact) or by performing a regression analysts. From that relationship, tensile strength at a standardized value of solid fraction can be estimated. The standardized value selected is one that is representative of the solid fraction of a desired sustained-release tablet, *e.g.*, 0.8.

Where the material of the compact is pregelatinized starch, it has been found that tensile strength as determined in a simple test as described immediately above is surprisingly close to a "true" tensile strength measurement as determined by the triaxial tensile strength test method previously described, which in turn is essentially similar to methods known in the art such as that disclosed by Hiestand & Smith (1984), *op*, *cit*.

It has also been found that a longer dwell time (*e.g.*, 90 seconds in the test method of the present invention gives a better correlation with triaxial tensile strength than a very short dwell time (*e.g.*, 4 seconds). See Example 1 below and Figs. 1 and 2.

Thus, a suitable method for determining suitability of a starch for use in a sustained-release orally delilverable tablet of the present invention comprises the steps of

(a) preparing compacts of a starch sample on an aged tablet press at a range of compression forces applied for a dwell time of at least about 4 seconds;
(b) measuring hardness of each compact, expressed as the force required to cause crushing ofthe compact;
(c) determining solid fraction of each compact;
(d) calculating tensile strength $\sigma_T$ of each compact from the equation

$$\sigma_T = 2F/\pi DH$$

where F is the force required to cause crushing, D is diameter of the compact and H is thickness of the contact;
(e) establishing relationship of tensile strength to solid fraction of the compacts, for example by plotting these parameters on a graph and/or by performing a regression analysis; and
(f) using the relationship established in step (e), estimating tensile strength at a solid traction representative of a desired sustained-release tablet; for example a solid traction of 0.8.

If tensile strength of the starch as so estimated is at least 0.15 kN $cm^{-2}$, the starch is deemed suitable for use.

An especially preferred starch has a tensile strength of at least 0.175 kN $cm^{-2}$, even more preferably at least 0.2 kN $cm^{-2}$, at a solid fraction representative of a desired sustained-release tablet.

Even among commercially available pregelatinized starches, the preferred type of starch for use in a composition of the invention, considerable variation exists in tensile strength. Pregelatinized starches not meeting the tensile strength criterion established herein are not readily identified without testing, for example by a method as disclosed above. Such pregelatinized starches are generally unsuitable for commercial scale manufacture of a sustained-release matrix tablet formulation of a water-soluble drug or prodrug, because of a problem as set forth immediately below.

An uncoated tablet, or a tablet core prior to coating, comprising starch and a hydrophilic polymer acting as a matrix for a water-soluble drug or pro drug requires to have a certain minimum hardness in order to be able to resist breakage and/or attrition due to mechanical stresses imposed during a high-speed tableting operation (including all steps up to and including filling of the tablets into containers). The minimum acceptable hardness will depend on a number of factors, including the severity of the mechanical stresses, but is typically at least 20 SCU, preferably at least 22 SCU, more preferably at least 24 SCU (about 17 kp).

Hardness can be increased by increasing the compression force applied by the tablet press, but only up to a certain level. At least in the case of tablets as described herein, above a certain compression force, further increases in compression force give little or not further increase in tablet hardness. There is, in other words, a maximum hardness achievable by compression of a particular starch/hydrophilic polymer/active agent composition. A starch providing a maximum hardness inadequate to withstand the mechanical stresses of a high-speed tableting operation is unsuitable for the present purpose. As shown in Fig. 3, certain pregelatinized starches have been found to provide a maximum hardness of 20 SCU or less; these are now identified as starches having low tensile strength (0.1 kN $cm^2$ or less according to the test method of the invention utilizing a dwell time of 90 seconds).

Even if a maximum hardness of at least 20 SCU is achievable, with a starch of low tensile strength it may be achievable only by use of extremely high compression forces. A requirement for such forces reduces speed and efficiency and increases cost of a tableting operation and is undesirable for these reasons.

Where tablets are to be subjected to an additional process step after compression, in particular a coating step, exposure to mechanical stresses is greatly increased. According to a preferred embodiment, therefore, the sustained-release tablet of the invention further comprises a coating.

For drugs and prodrugs of high water solubility as specified herein, a hydrophilic polymer matrix is often inadequate to provide sustained release of sufficiently long duration to permit once daily administration. It is believed that such drugs are readily leached out of the hydrophilic matrix when contacted by an aqueous medium such as gastrointestinal fluid. It is therefore desirable to further slow the process of drug release by providing a release-controlling coating around the tablet. Such a coating typically comprises a hydrophobic or water-insoluble polymer component such as ethylcellulose together with a hydrophilic or water-soluble pore-forming component such as HPMC.

Where a starch is used having a tensile strength of at least 0.15 kN $cm^{-2}$, preferably at least 0.175 kN $cm^{-2}$, more preferably at least 0.2 kN $cm^{-2}$, at a solid fraction representative of the tablet (*e.g.,* 0.75 to 0.85), the composition is found to be especially suited to a high-speed tableting operation that includes a step of coating the tablet with a release-controlling layer.

Alternatives to ethylcellulose and HPMC as components of a release coating layer include other cellulosic polymers (*e.g.*, methylcellulose, hydroxypropylcellulose, hydroxyethylcellulose, carboxymethylcellulose sodium, cellulose esters such as cellulose acetate, *etc.*), polyvinyl acetate, polyvinyl pyrrolidone, polymers and copolymers of acrylic acid and methacrylic acid and esters thereof, polyethylene glycol, carrageenan and other gums, and the like.

A release-controlling layer, if present, typically constitutes 1% to 15%, preferably 2.5% to 10%, by weight of the tablet as a whole. The hydrophobic or water-insoluble component, preferably comprising ethylcellulose, typically constitutes 1% to 10%, preferably 2% to 7%, by weight of the tablet as a whole. The pore-forming component, preferably comprising HPMC, is typically present in an amount of 5% to 50%, preferably 10% to 40%, by weight of the water-insoluble or hydrophobic component.

The coating, if present, can optionally contain additional pharmaceutically acceptable excipients such as plasticizers, dyes, *etc*.

Illustratively, a release-controlling layer in an amount of 2.5% to 5% by weight of the tablet core (*i.e.*, the tablet weight excluding the coating) comprises an ethylcellulose-based material (*e.g.*, Surelcase® of Colorcon) and an HPMC-based pore-forming material (*e.g.*, Opadry® of Colorcon) in a weight ratio of 3:1 to 4:1.

A release-controlling layer or coating should be applied at as uniform a thickness as possible to provide optimum control of release rate of the active pharmaceutical agent.

Alternatively or in addition, the sustained-release tablet of the invention comprises a nonfunctional coating. A nonfunctional coating can comprise a polymer component, for example HPMC, optionally with other ingredients, for example one or more plasticizers, colorants, *etc*. The term "nonfunctional" in the present context means having no substantial effect on release properties of the tablet, and does not imply that the coating serves no useful purpose. For example, such a coating can impart a distinctive appearance to the tablet, provide protection against attrition during packaging and transportation, improve ease of swallowing, and/or have other benefits. A nonfunctional coating should be applied in an amount sufficient to provide complete coverage of the tablet. Typically an amount of 1% to 10%, more

typically an amount of 2.5% to 5%, by weight of the tablet as a whole, will be found suitable.

Uncoated tablets and cores of coated tablets of the invention optionally contain one or more pharmaceutically acceptable excipients in addition to the starch and hydrophilic polymer components described above. Such excipients include without limitation glidants and lubricants. Other conventional excipients known in the art can also be included.

A glidant can be used to improve powder flow properties prior to and during tableting and to reduce caking. Suitable glidants include colloidal silicon dioxide, magnesium trisilicate, powdered cellulose, starch, talc, tribasic calcium phosphate and the like. In one embodiment, colloidal silicon dioxide is included as a glidant in an amount up to 2%, preferably 0.2% to 0.6%, by weight of the tablet.

A lubricant can be used to enhance release of a tablet from apparatus on which it is formed, for example by preventing adherence to the face of an upper punch ("picking") or lower punch ("stricking"). Suitable lubricants include magnesium stearate, calcium stearate, canola oil, glyceryl palmitostearate, hydrogenated vegetable oil, magnesium oxide, mineral oil, poloxamer, polyethylene glycol, polyvinyl alcohol, sodium benzoate, sodium lauryl sulfate, sodium stearyl fumarate, stearic acid, talc, hydrogenated vegetable oil zinc stearate and the like. In one embodiment, magnesium stearate is included as a lubricant in an amount of 0.1 % to 1.5%, preferably 0.3% to 1%, by weight of the tablet,

Tablets can be of any suitable size and shape, for example round, oval, polygonal or pillow-shaped, and optionally bear nonfunctional surface makings, Especially in the case of coated tablets they are preferably designed to be swallowed whole and are therefore typically not provided with a breaking score. Tablets of the invention can be packaged in a container, accompanied by a package insert providing pertinent information such as, for example, dosage and administration information, contraindications, precautions, drug interactions and adverse reactions. Compositions of the present invention can be used to treat CNS conditions or disorders.

In one embodiment, the condition of disorder is a CNS condition or disorder and the agent is a CNS agent as defined herein. CNS conditions and disorders include those having a neurologic and/or a psychiatric component.

Illustrative CNS conditions and disorders treatable by the method of the invention include, for example, personality disorders including paranoid, schizoid, schizotypal, bipolar, histrionic, delusional, narcissistic, emotionally unstable, psychopathic and sociopathic personality disorders; habit and impulse disorders including pathological gambling, stealing, trichotillomania, *etc.*; obsessive-compulsive disorder; passive-aggressive disorder; acute and transient psychotic disorders; psychotic depression; schizoaffective disorder; hypochondria; cyclothymia; dysthymia; manic-depressive illness; major depressive disorder; treatment-resistant depression; adult and childhood onset schizophrenias; drug dependence including harmful use and abuse of, addiction to or dependence on opioids, narcotics, barbiturates, alcohol, benzodiazepines, amphetamines, cocaine, cannabinoids, hallucinogens, stimulants, nicotine (tobacco) and solvents; withdrawal states and mood and psychotic disorders related to such dependence; sexual dysfunction including hypoactive sexual desire disorder, sexual aversion or avoidance and erectile dysfunction; gender identity disorders; sexual preference disorders; general anxiety disorder; social anxiety disorder, mixed anxiety and depressive disorder, attention deficit hyperactivity disorder (ADHD) and depression and anxiety associated therewith; depression, anxiety, emotional dysregulation and behavioral disturbances associated with mental retardation; developmental disorders including autism, Asperger's syndrome and Rett's syndrome; childhood conduct and attachment disorders; premenstrual dysphoric disorder; postpartum depression; phobias including social phobias, agoraphobia and specific phobias related for example to hospitals, injections, venesection, *etc.*; posttraumatic stress disorder; dissociative disorder; Briquet's syndrome; effective disorders including depression, bipolar affective disorder and recurrent depressive disorder; organic mood, anxiety and emotionally labile disorders resulting for example from brain damage or dysfunction arising from head injury, intracranial masses, stroke, etc.; chronic fatigue; stress-induced psychotic episodes; dementia including presenile dementia, Pick's disease, vascular dementia, multi-infarct dementia, Alzheimer's disease, dementia associated with Creutzfeldt-Jakob disease and HIV-related dementia; other neurodegenerative disorders including Parkinson's disease and Huntington's disease; suicidal behavior; cating disorders including anorexia, bulimia and binge eating disorder; adjustment disorders; somatization disorder; somatoform autonomie dysfunction; somatoform pain disorder; panic attacks; panic disorder; amnesia; neuropathic pain; fibromyalgia; migraine; epilepsy; tinnitus; enuresis; sleep disorders including insomnia, hypersomnia, narcolepsy, nightmares and night terrors; delirium; postconcussion syndrome; multiple sclerosis; tremors; muscular spasms; restless legs syndrome; Lennox-Gastaut syndrome; motor and vocal tic disorders; Tourette's syndrome; supranuclear palsy; Shy-Drager syndrome; trigeminal neuralgia; Bell's palsy; motor neuron diseases such as amyotrophic lateral sclerosis; and psychosomatic and psychosocial conditions associated with non-CNS diseases such as diabetes, inflammatory disease, infertility, allergies, psoriasis, asthma, hypertension, overactive bladder, thyroid disorders, obesity, immune disorders and cancer.

In a particular embodiment, the condition or disorder is one that is responsive to dopamine $D_2$ receptor agonist therapy or to SNRI therapy, and the active pharmaceutical agent is a dopamine $D_2$ receptor agonist or SNRI or prodrug thereof. Presently preferred SNRIs for use according to the method of the invention include salts of reboxetine and (S, S)-reboxetine. Such SNRIs are particularly useful in treatment of depressive illness and neuropathic pain, including postherpetic neuralgia and diabetic neuropathy. In the case of reboxetine or (S,S)-reboxetine, suitable daily dosage amounts include 1, 2, 4, 6, 8 and 12 mg reboxetine in the form ofits mesylate salt or (S,S)-reboxetine in the form of its

succinate salt.

In a further embodiment, a composition of the invention is administered in combination therapy with one or more additional drugs or prodrugs. The term "combination therapy" herein means a treatment regimen wherein the agent provided by the composition of the invention and a second agent are administered individually or together, sequentially or simultaneously, in such a way as to provide a beneficial effect from co-action of these therapeutic agents. Such beneficial effect can include, but is not limited to, pharmacokinetic or pharmacodynamic co-action of the therapeutic agents. Combination therapy can, for example, enable administration of a lower dose of one or both agents than would normally be administered during monotherapy, thus decreasing risk or incidence of adverse effects associated with higher doses. Alternatively, combination therapy can result in increased therapeutic effect at the normal dose of each agent in monotherapy. "Combination therapy" herein is not intended to encompass administration of two or more therapeutic agents as part of separate monotherapy regimens that incidentally and arbitrarily result in sequential or simultaneous treatment.

Compositions of the invention can be especially suited to combination therapies, particularly where the second agent is one that is, or can be, administered once daily. There are significant advantages in patient convenience and compliance where both components of a combination therapy can be administered at the same time and with the same frequency. This is especially true in the case of geriatric patients or those suffering memory impairment.

When administered simultaneously, the two components of the combination therapy can be administered in separate dosage forms or in coformulation, *i.e.*, in a single dosage form. When administered sequentially or in separate dosage forms, the second agent can be administered by any suitable route and in any pharmaceutically acceptable dosage form, for example by a route and/or in a dosage form other than the present composition. In a preferred embodiment, both components of the combination therapy are formulated together in a single dosage form.

An illustrative combination therapy involves once daily administration of a composition of the invention comprising an SNRI, for example a salt ofreboxetine or (S,S)-reboxetine, and once daily administration of an SSRI, for example fluoxetine, fluvoxamine, paroxetine or sertraline or a salt thereof SNRI/SSRI combination therapies have been proposed, *e.g.*, for treatment-resistant depression as disclosed by Forbes & Rogers (2003), Progress in Neurology and Psychiatry 7(2), 10-14; according to the present invention both components of such a combination therapy can be administered once daily, with concomitant improvement in patient compliance.

## EXAMPLES

### Example 1

Tensile strength of six commercially obtained lots of pregelatinized starch was determined using the triaxial tensile strength test procedure described hereinabove. Data for tensile strength at a solid fraction of 0.8 are presented in Table 1.

**Table 1. Tensile strength of pregelatinized starch lots at a solid fraction of 0.8 (triaxial test procedure)**

| Lot | Tensile strength (kN $cm^{-2}$) |
|---|---|
| 1 | 0.323 |
| 2 | 0.220 |
| 3 | 0.074 |
| 4 | 0.119 |
| 5 | 0.287 |
| 6 | 0.236 |

A great variation in tensile strength of pregelatinized starches was observed, ranging from 0.074 to 0.323 kN $cm^{-2}$. Lots 3 and 4, exhibiting the lowest values of tensile strength, were from one manufacturer. Lots 1, 5 and 6, exhibiting the highest values of tensile strength, were from a second manufacturer. Lot 2, exhibiting an intermediate value of tensile strength, was from a third manufacturer.

### Example 2

Tensile strength of the same six lots of pregelatinized starch was determined by the following simplified test procedure.

Compacts of each starch lot were prepared on a Carver press, Model 3888.1DT0000 fitted with 10/32 inch (0.7 cm) flat-faced tooling, at compression forces of 1000, 1500, 2000 and 3000 lbf (4.45, 6.67, 8.90 and 13.34 kN), for a dwell time of 4 seconds or 90 seconds. Compacts of an additional three lots of pregelatinized starch (Lots 7, 8 and 9), from the same manufacturer as Lots 3 and 4, were prepared using a dwell time of 90 seconds only. Weight and thickness of each compact was measured (diameter being equal to that of the tooling) to enable calculation of apparent density. Absolute density of each starch lot was measured by helium-air pycnometry. Solid fraction was calculated as the ratio of apparent to absolute density.

Hardness (force required to cause crushing) of each compact was determined using a Key HT 500 hardness tester. Tensile strength was calculated from this force and dimensions of the compact, using the equation

$$\sigma_T = 2F/\pi DH$$

as described hereinabove.

A regression analysis was performed to determine the relationship of tensile strength to solid fraction for each starch lot, and tensile strength at a standardized solid fraction of 0.8 was calculated. Data are presented in Table 2.

**Table 2. Tensile strength of pregelatinized starch lots at a solid fraction of 0.8 (simplified test procedure of the invention)**

| Lot | Tensile strength (kN $cm^{-2}$) | |
|---|---|---|
| | 4 s dwell time | 90 s dwell time |
| 1 | 0.310 | 0.306 |
| 2 | 0.227 | 0.191 |
| 3 | 0.092 | 0.085 |
| 4 | 0.134 | 0.096 |
| 5 | 0.316 | 0.277 |
| 6 | 0.333 | 0.242 |
| 7 | n.d | 0.087 |
| 8 | n.d. | 0.088 |
| 9 | n.d. | 0.172 |

Correlation of tensile strength as measured in the simplified test using a 4 second dwell time (this Example) with tensile strength as treasured by the triaxial test procedure of Example 1 is shown graphically in Fig. 1.

Correlation of tensile strength as measured in the simplified test using a 90 second dwell time (this Example) with tensile strength as measured by the triaxial test procedure of Example 1 is shown graphically in Fig. 2.

Both dwell times exhibited a strong correlation, but the correlation was especially close where the simplified test used a 90 second dwell time. It is concluded that the simplified test as herein described can be used to estimate tensile strength of a starch lot for the purpose of predicting whether that starch lot will be suitable for preparing a sustained-release tablet formulation of the present invention.

<u>Reference Example 3</u>

Sumanirole maleate sustained-release tablets were prepared having the compositions shown in Table 3. Tablet strength in mg is expressed as sumanirole base.

**Table 3. Composition of sumanirole maleate tablets of Example 3**

| Ingredient | Tablet strength (mg) | | | | | | | |
|---|---|---|---|---|---|---|---|---|
| | 0.5 | 1 | 2 | 4 | 8 | 8 | 12 | 24 |
| | Amount (% by weight) | | | | | | | |
| sumanirole maleate | 0.23 | 0.45 | 0.9 | 1.8 | 3.6 | 3.6 | 5.4 | 10.9 |
| HPMC type 2208, 4000 mPa s | 35.00 | 35.00 | 35.0 | 35.0 | 35.0 | 35.0 | 35.0 | 35.0 |
| pregelatinized starch | 63.87 | 63.65 | 63.2 | 62.3 | 60.5 | 60.0 | 58.2 | 52.5 |
| colloidal silicon dioxide | 0.40 | 0.40 | 0.4 | 0.4 | 0.4 | 0.4 | 0.4 | 0.4 |
| magnesium stearate | 0.50 | 0.50 | 0.5 | 0.5 | 0.5 | 1.0 | 1.0 | 1.0 |

All ingredients except the lubricant (magnesium stearate) were screened to remove lumps and were mended thoroughly in a low-shear mixes operating at 24 rpm for 10-30 minutes. The lubricant was then screened into the mixer and the materials were blended for a further 2-5 minutes. The resulting lubricated mixture was compressed into 350 mg pillow-shaped tablets using a Kilian S100 tableting machine.

Reference Example 4

Tablets similar to those of Example 3 were prepared using pregelatinized starches of lots 1-6 as tested in Examples 1 and 2. Maximum hardness of the tablets obtainable with each pregelatinized starch lot was determined.

Maximum hardness was correlated with tensile strength of the pregelatinized starch lot used, as measured in the simplified test of Example 2 using a 90 second dwell time. Results are shown in Fig. 3. The correlation was substantially linear.

In subsequent tests, tablets of different hardness were used as cores for coating and were tested for resistance to erosion during a high-speed coating operation. Tablet cores having a hardness of at least about 24 SCU (about 17 kp) were found to have acceptable resistance to erosion. As shown in Fig. 3, this degree of hardness is achievable using pragelatinized starch having a tensile strength of at least about 0.175 kN $cm^{-2}$. Pregelatinized starches of Lots 3 and 4 were unsuitable, having tensile strength less than about 0.15 kN $cm^{-2}$ and providing tablets having a maximum hardness no greater than about 20 SCU (about 14 kp).

Reference Example 5

Pramipexole dihydrochloride sustained-release tablets were prepared having the compositions shown in Table 4.

**Table 4. Composition of pramipexole dihydrochloride tablets of Example 5**

| Ingredient | Amount (mg) | | | | | | | |
|---|---|---|---|---|---|---|---|---|
| pramipaxole dihydrochloride monohydrate | 0.375 | 0.75 | 1.5 | 3.0 | 4.5 | 0.375 | 0.375 | 4.5 |
| HPMC type 2208, 4000 mPa s | 140.0 | 140.0 | 140.0 | 140.0 | 140.0 | 70.0 | 157.5 | 157.5 |
| pregelatinized starch | 206.5 | 206.1 | 205.4 | 203.9 | 202.4 | 101.5 | 189.0 | 184.9 |
| colloidal silicon dioxide | 1.4 | 1.4 | 1.4 | 1.4 | 1.4 | 1.4 | 1.4 | 1.4 |
| magnesium stearate | 1.75 | 1.75 | 1.75 | 1.75 | 1.75 | 1.75 | 1.75 | 1.75 |
| total | 350 | 350 | 350 | 350 | 350 | 175 | 350 | 350 |

The tablets were prepared by the procedure described in Example 3, using pregelatinized starch having a tensile strength of at least about 0.175 kN $cm^{-2}$.

Reference Example 6

Coated sustained-release tablets of pramipexole dihydrochloride were prepared having the composition shown

in Table 5,

**Table 5, Composition of coated tablets of Example 6**

| Ingredient | Amount (mg) |
|---|---|
| pramipexole dihydrochloride monohydrate | 0.375 |
| HPMC type 2208, 4000 mPas | 140.0 |
| pregelatinized starch | 206.5 |
| colloidal silicon dioxide | 1.4 |
| magnesium stearate | 1.75 |
| total core | 350 |
| coating material (Surelease®) | 7.88 |
| HPMC-based coating material (Opadry®) | 2.63 |
| total coating | 10.5 |

Tablet cores were prepared exactly as in Example 5, using pregelatinized starch having a tense strength of at least about 0.175 kN $cm^{-2}$. A coating solution was prepared as follows. Opadry® HPMC-based material in an amount of 6.004 g was added to 106.652 g water and mixed for 45 minutes to provide an HPMC mixture. Next, 72.045 g Surelease® ethylcellulose-based material was added to the HPMC mixture and mixed for an additional 30 minutes to provide a coating solution.

The coating solution was applied to the tablet cores in an amount providing a 3% weight gain. The resulting coated tablets were cured using a 12 inch (about 30 cm) Vector LCDS or 24 inch (about 60 cm) Thomas Accela-Coata coating pan for about 15 minutes at a bed temperature of at least about 70°C, After curing, temperature was ramped down over a period of about 8 minutes to an exhaust temperature of about 45°C.

Reference Example 7

Coated sustained-release tablets of pramipexole dihydrochloride were prepared having the composition shown in Table 6.

**Table 6. Composition of caoted tablets of Example 7**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 8.4 |
| HPMC-based coating material (Opadry®) | 2.1 |
| total coating | 10.5 |

Tablet cores were prepared exactly as in Example 5, using pregelatinized starch having a tensile strength of at least about 0.175 kN $cm^{-2}$ , A coating solution was prepared as follows. Opadry® HPMC-based material in an amount of 4.801 g was added to 103.041 g water and mixed for 45 minutes to provide an HPMC mixture. Next, 76.819 g Surelease® ethylcellulose-based material was added to the HPMC mixture and mixed for an additional 30 minutes to provide a coating solution.

Coating to a 3% weight gain and curing of the coated tablets were performed exactly as in Example 6.

Reference Example 8

Coated sustained-release tablets of pramipexole dihydrochloride were prepared having the composition shown in Table 7.

**Table 7. Composition of coated tablets of Example 8**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 13.13 |
| HPMC-based coating material (Opadry®) | 4-38 |
| total coating | 17.5 |

Tablet cores were prepared exactly as in Example 5, using pregelatinized starch having a tensile strength of at least about 0.175 kN $cm^{-2}$. A coating solution was prepared as follows. Opadry® HPMC-based material in an amount of 10.003 g was added to 177.737 g water and mixed for 45 minutes to provide an HPMC mixture. Next, 120.03 g Surolease® etylcellulose-based material was added to the HPMC mixture and mixed for an additional 30 minutes to provide a coating solution.

Coating to a 3% weight gain and curing of the coated tablets were performed exactly as in Example 6. After this first curing step, coating was repeated to provide a total tablet weight gain of about 5%, followed by curing for about 15 minutes at a bed temperature of at least about 70°C. After curing, temperature was ramped down over a period of about 8 minutes to an exhaust temperature of about 45°C.

Reference Example 9

Coated sustained-release tablets of pramipexole dihydrochloride were prepared having the composition shown in Table 8.

**Table 8. Composition of coated tablets of Example 9**

| Ingredient | Amount (mg) |
|---|---|
| pramipexole dihydrochloride monohydrate | 0.375 |
| HPMC type 2208, 4000 mPa s | 140.0 |
| pregelatinized starch | 206.5 |
| colloidal silicon dioxide | 1.4 |
| magnesium stearate | 1.75 |
| total core | 350 |
| ethylcellulose-based coating material (Surelease®) | 14.0 |
| HPMC-based coating material (Opadry®) | 3.5 |
| total coating | 17.5 |

Tablet cores were prepared exactly as in Example 5, using pregelatinized starch having a tensile strength of at least about 0.175 kN $cm^{-2}$. A coating solution was prepared as follows. Opadry® HPMC-based material in an amount of 8.002 g was added to 171.735 g water and mixed for 45 minutes to provide an HPMC mixture. Next, 128,032 g Surelease® ethylcellulose-based material was added to the HPMC mixture and mixed for an additional 30 minutes to

provide a coating solution.

Coating to a 5% total weight gain and curing of the coated tablets were performed exactly as in Example 8.

Reference Example 10

Dissolution profiles of the 0.375 mg pramipexole dihydrochloride tablets of each of Examples 5, 6 and 9 were evaluated in a standard *in vitro* USP dissolution assay under the following conditions. USP apparatus 1 was used to stir a dissolution medium (900 ml of 0.05M phosphate buffer at a pH of 6.8) at a spindle rotation speed of 100 rpm and a temperature of 37°C.

Data are shown in Fig. 4. The uncoated tablet of Example 5 and the tablet of Example 6 having a 3% coating comprising 25% pore-former exhibited very similar overall dissolution profiles. On close inspection, however, it will be noticed that the uncoated tablet of Example 5 showed faster initial dissolution, such that at 1 hour and 2 hour sampling times the percent dissolved was greater, than in the case of the coated tablet of Example 6. For example, at 1 hour, the coated tablet of Example 6 showed only 11 % dissolution, while the uncoated tablet of Example 5 showed 15% dissolution. Similarly, at 2 hours, the coated tablet of Example 6 showed no more than 20% dissolution, while the uncoated tablet of Example 5 showed 24% dissolution.

Dissolution of the tablet of Example 9 having a 5% coating comprising 20% pore-former exhibited a dissolution profile much slower than either the tablet of Example 5 or the tablet of Example 6.

Example 11

(S,S)-reboxetine succinate sustained-release tablets were prepared having the compositions shown in Table 9. It will be noted that each tablet comprises 5.5 mg (S,S)-reboxetine succinate, equivalent to 4 mg (S,S)-reboxetine base.

**Table 9. Composition of (S,S)-reboxetine succinate tablets of Example 11**

| **Ingredient** | **Amount (mg)** | | |
|---|---|---|---|
| (S,S)-reboxetine succinate | 5.5 | 5.5 | 5.5 |
| HPMC type 2208, 4000 mPas | 40.0 | 80.0 | 160.0 |
| pregelatinized starch | 53.5 | 112.5 | 230.5 |
| colloidal silicon dioxide | 0.5 | 1.0 | 2.0 |
| magnesium stearate | 0.5 | 1.0 | 2.0 |
| total | 100.0 | 200.0 | 400.0 |

The tablets were prepared by the procedure described in Example 3, using pregelatinized starch having a tensile strength of at least about 0.175 kN $cm^{-2}$.

Example 12

Dissolution profiles of the 4 mg (S,S)-reboxetine tablets of Example 11 were evaluated in a standard *in vitro* USP dissolution assay under the following conditions. USP apparatus 2 was used to stir a dissolution medium (1 liter of 0.05M phosphate buffer at a pH of 6.8) at a paddle rotation speed of 50 rpm and a temperature of 37°C. The medium was then filtered and samples were analyzed via UV detection.

Data are shown in Fig. 5. The tablet having 100 mg total weight exhibited the fastest dissolution, and the tablet having 400 mg total weight the slowest. The tablet having 200 mg total weight was intermediate in dissolution rate.

**Claims**

1. A sustained-release pharmaceutical composition in a form of an orally deliverable tablet for treating a CNS condition or disorder comprising an active pharmaceutical agent which is reboxetine or a salt thereof, the active pharmaceutical ingredient being dispersed in a matrix comprising a hydrophilic polymer and a starch having a tensile strength of at least 0.15 kN $cm^{-2}$ at a solid fraction of 0.75 to 0.85 of the tablet.

2. The composition of Claim 1 wherein the starch has a tensile strength of at least 0.175 kN $cm^{-2}$, preferably a tensile

strength of at least 0.2 kN $cm^{-2}$ at a solid fraction of 0.75 to 0.85 of the tablet.

**3.** The composition of Claim 1 wherein the starch is a pregelatinized starch.

**4.** The composition of any of the preceding claims wherein the starch is present in an amount of 25% to 75%, preferably 40% to 70%, more preferably 45% to about 65%, by weight,

**5.** The composition of any of the preceding claims wherein the hydrophilic polymer is selected from the group consisting of methylcellulose, hydroxypropylmethylcellulose, carmellose sodium and carbomer.

**6.** The composition of any of the preceding claims wherein the hydrophilic polymer is hydroxypropylmethylcellulose.

**7.** The composition of any of the preceding claims wherein the hydrophilic polymer is present in an amount of 20% to 70%, preferably 30% to 60%, more preferably 35% to 50%, by weight.

**8.** The composition of any of the preceding claims wherein the active pharmaceutical agent is (S, S)-reboxetine or a salt thereof.

**9.** The composition of Claim 8 wherein the active pharmaceutical agent is (S,S)-reboxetine succinate.

**10.** The composition of Claim 8 or Claim 9 that comprises 0.2 to 15 mg, preferably 1 to 12 mg reboxetine per tablet.

**11.** The composition of any of the preceding claims, further comprising a coating on the tablet.

**12.** The composition of Claim 11 wherein said coating is a release-controlling layer.

**13.** The composition of Claim 12 wherein said release-controlling layer constitutes 1% to 15% by weight of the tablet.

**14.** The composition of Claim 11 wherein said coating is a nonfunctional coating.

**15.** A pharmaceutical composition according to Claim 1, in a form of an orally deliverable tablet, comprising (S, S) reboxetine succinate in an amount of 1, 2, 4, 6, 8 or 12 mg base equivalent, dispersed in a matrix comprising (a) HPMC type 2208 in an amount of 35% to 50% by weight of the tablet and (b) a pre-gelatinised starch having a tensile strength of at least 0.15 kN $cm^{-2}$ at a solid fraction of 0.8, in an amount of 45% to 65% by weight of the tablet.

**16.** Use of the composition of Claim 1 in the manufacture of a medicament for treating a CNS condition or disorder selected from the group consisting of psychotic depression; manic-depressive illness; major depressive disorder; treatment-resistant depression; neuropathic pain; fibromyalgia; and migraine.

**17.** A process for preparing a sustained-release pharmaceutical composition according to Claim 1 in a form of an orally deliverable tablet, the process comprising selecting by a suitable test a starch having a tensile strength of at least 0.15 kN $cm^{-2}$ at a solid fraction of 0.75 to 0.85 of the tablet; admixing with the selected starch a hydrophilic polymer and an active pharmaceutical agent which is reboxetine or a salt thereof, to provide a mixture wherein the agent is dispersed in a matrix comprising the polymer and the starch; and compressing the mixture to form said tablet.

**Patentansprüche**

**1.** Freisetzungsverzögerte pharmazeutische Zusammensetzung in Form einer oral verabreichbaren Tablette zum Behandeln eines/einer ZNS-Zustands oder -Erkrankung, umfassend ein aktives pharmazeutisches Mittel, das Reboxetin oder ein Salz davon ist, wobei das aktive pharmazeutische Mittel in einer Matrix dispergiert ist, umfassend ein hydrophiles Polymer und eine Stärke mit einer Zugfestigkeit von wenigstens 0,15 kN $cm^{-2}$ bei einem Feststoffanteil von 0,75 bis 0,85 der Tablette.

**2.** Zusammensetzung nach Anspruch 1, wobei die Stärke eine Zugfestigkeit von wenigstens 0,175 kN $cm^{-2}$, vorzugsweise eine Zugfestigkeit von wenigstens 0,2 kN $cm^{-2}$ bei einem Feststoffanteil von 0,75 bis 0,85 der Tablette hat.

**3.** Zusammensetzung nach Anspruch 1, wobei die Stärke eine vorverkleisterte Stärke ist.

4. Zusammensetzung nach einem der vorstehenden Ansprüche, wobei die Stärke in einer Menge von 25 Gew.-% bis 75 Gew.-%, vorzugsweise 40 Gew.-% bis 70 Gew.-%, stärker bevorzugt 45 Gew.-% bis etwa 65 Gew.-% vorliegt.

5. Zusammensetzung nach einem der vorstehenden Ansprüche, wobei das hydrophile Polymer ausgewählt ist aus der Gruppe, bestehend aus Methylcellulose, Hydroxypropylmethylcellulose, Carmellose-Natrium und Carbomer.

6. Zusammensetzung nach einem der vorstehenden Ansprüche, wobei das hydrophile Polymer Hydroxypropylmethylcellulose ist.

7. Zusammensetzung nach einem der vorstehenden Ansprüche, wobei das hydrophile Polymer in einer Menge von 20 Gew.-% bis 70 Gew.-%, vorzugsweise 30 Gew.-% bis 60 Gew.-%, stärker bevorzugt 35 Gew.-% bis 50 Gew.-% vorliegt.

8. Zusammensetzung nach einem der vorstehenden Ansprüche, wobei das aktive pharmazeutische Mittel (S,S)-Reboxetin oder ein Salz davon ist.

9. Zusammensetzung nach Anspruch 8, wobei das aktive pharmazeutische Mittel (S,S)-Reboxetinsuccinat ist.

10. Zusammensetzung nach Anspruch 8 oder Anspruch 9, die 0,2 bis 15 mg, vorzugsweise 1 bis 12 mg Reboxetin pro Tablette umfasst.

11. Zusammensetzung nach einem der vorstehenden Ansprüche, die des Weiteren einen Überzug auf der Tablette umfasst.

12. Zusammensetzung nach Anspruch 11, wobei der Überzug eine freisetzungskontrollierende Schicht ist.

13. Zusammensetzung nach Anspruch 12, wobei die freisetzungskontrollierende Schicht 1% bis 15%, bezogen auf das Gewicht der Tablette, ausmacht.

14. Zusammensetzung nach Anspruch 11, wobei der Überzug ein nichtfunktioneller Überzug ist.

15. Pharmazeutische Zusammensetzung nach Anspruch 1 in Form einer oral verabreichbaren Tablette, umfassend (S, S)-Reboxetinsuccinat in einer Menge von 1, 2, 4, 6, 8 oder 12 mg Basenäquivalent, dispergiert in einer Matrix, umfassend (a) HPMC Typ 2208 in einer Menge von 35% bis 50%, bezogen auf das Gewicht der Tablette, und (b) eine vorverkleisterte Stärke mit einer Zugfestigkeit von wenigstens 0,15 kN $cm^{-2}$ bei einem Feststoffanteil von 0,8, in einer Menge von 45% bis 65%, bezogen auf das Gewicht der Tablette.

16. Verwendung der Zusammensetzung nach Anspruch 1 bei der Herstellung eines Medikaments zum Behandeln eines/einer ZNS-Zustands oder -Erkrankung, ausgewählt aus der Gruppe, bestehend aus psychotischer Depression, manisch-depressiver Krankheit; schwerer depressiver Störung; Therapie-resistenter Depression; neuropathischem Schmerz; Fibromyalgie und Migräne.

17. Verfahren zum Herstellen einer freisetzungsverzögerten pharmazeutischen Zusammensetzung nach Anspruch 1 in Form einer oral verabreichbaren Tablette, wobei das Verfahren umfasst: Auswählen einer Stärke mit einer Zugfestigkeit von wenigstens 0,15 kN $cm^{-2}$ bei einem Feststoffanteil von 0,75 bis 0,85 der Tablette durch einen geeigneten Test; Mischen der ausgewählten Stärke mit einem hydrophilen Polymer und einem aktiven pharmazeutischen Mittel, das Reboxetin oder ein Salz davon ist, um ein Gemisch bereitzustellen, worin das Mittel in einer Matrix dispergiert ist, die das Polymer und die Stärke umfasst; und Pressen des Gemischs, um die Tablette zu formen.

## Revendications

1. Composition pharmaceutique à libération prolongée sous forme d'un comprimé apte à l'administration orale pour le traitement d'une affection ou d'un trouble du système nerveux central (SNC), comprenant un agent pharmaceutique actif qui est la réboxétine ou un de ses sels, l'ingrédient pharmaceutique actif étant dispersé dans une matrice comprenant un polymère hydrophile et un amidon ayant une résistance à la traction d'au moins 0,15 kN $cm^{-2}$ à une fraction en matières solides de 0,75 à 0,85 du comprimé.

**2.** Composition suivant la revendication 1, dans laquelle l'amidon a une résistance à la traction d'au moins 0,175 kN $cm^{-2}$, de préférence une résistance à la traction d'au moins 0,2 kN $cm^{-2}$ à une fraction en matières solides de 0,75 à 0,85 du comprimé.

**3.** Composition suivant la revendication 1, dans laquelle l'amidon est un amidon prégélatinisé.

**4.** Composition suivant l'une quelconque des revendications précédentes, dans laquelle l'amidon est présent en une quantité de 25 % à 75 %, avantageusement de 40 % à 70 %, plus avantageusement de 45 % à environ 65 %, en poids.

**5.** Composition suivant l'une quelconque des revendications précédentes, dans laquelle le polymère hydrophile est choisi dans le groupe consistant en la méthylcellulose, l'hydroxypropylméthylcellulose, le carmellose sodique et un carbomère.

**6.** Composition suivant l'une quelconque des revendications précédentes, dans laquelle le polymère hydrophile est l'hydroxypropylméthylcellulose.

**7.** Composition suivant l'une quelconque des revendications précédentes, dans laquelle le polymère hydrophile est présent en une quantité de 20 % à 70 %, avantageusement de 30 % à 60 %, plus avantageusement de 35 % à 50 %, en poids.

**8.** Composition suivant l'une quelconque des revendications précédentes, dans laquelle l'agent pharmaceutique actif est la (S,S)-réboxétine ou un de ses sels.

**9.** Composition suivant la revendication 8, dans laquelle l'agent pharmaceutique actif est le succinate de (S,S)-réboxétine.

**10.** Composition suivant la revendication 8 ou la revendication 9, qui comprend 0,2 à 15 mg, de préférence 1 à 12 mg, de réboxétine par comprimé.

**11.** Composition suivant l'une quelconque des revendications précédentes, comprenant en outre un enrobage sur le comprimé.

**12.** Composition suivant la revendication 11, dans laquelle ledit enrobage est une couche commandant la libération.

**13.** Composition suivant la revendication 12, dans laquelle ladite couche commandant la libération représente 1 % à 15 % en poids du comprimé.

**14.** Composition suivant la revendication 11, dans laquelle ledit enrobage est un enrobage non fonctionnel.

**15.** Composition pharmaceutique suivant la revendication 1, sous forme d'un comprimé pouvant être administré par voie orale, comprenant du succinate de (S,S)-réboxétine en une quantité de 1, 2, 4, 6, 8 ou 12 mg d'équivalent de base, à l'état dispersé dans une matrice comprenant (a) de la HPMC de type 2208 en une quantité de 35 % à 50 % en poids du comprimé et (b) un amidon prégélatinisé ayant une résistance à la traction d'au moins 0,15 kN $cm^{-2}$ à une fraction en matières solides de 0,8, en une quantité de 45 % à 65 % en poids du comprimé.

**16.** Utilisation de la composition de la revendication 1 dans la production d'un médicament destiné au traitement d'une affection ou d'un trouble du SNC choisi dans le groupe consistant en la dépression psychotique ; la maladie dépressive maniaque ; un trouble dépressif majeur ; la dépression résistante au traitement ; la douleur neuropathique ; la fibromyalgie ; et la migraine.

**17.** Procédé pour la préparation d'une composition pharmaceutique à libération prolongée suivant la revendication 1, sous forme d'un comprimé pouvant être administré par voie orale, le procédé comprenant la sélection par un test convenable d'un amidon ayant une résistance à la traction d'au moins 0,15 kN $cm^{-2}$ à une fraction en matières solides de 0,75 à 0,85 du comprimé ; le mélange à l'amidon choisi d'un polymère hydrophile et d'un agent pharmaceutique actif qui est la réboxétine ou un des ses sels, pour fournir un mélange dans lequel l'agent est dispersé dans une matrice comprenant le polymère et l'amidon ; et la compression du mélange pour former ledit comprimé.

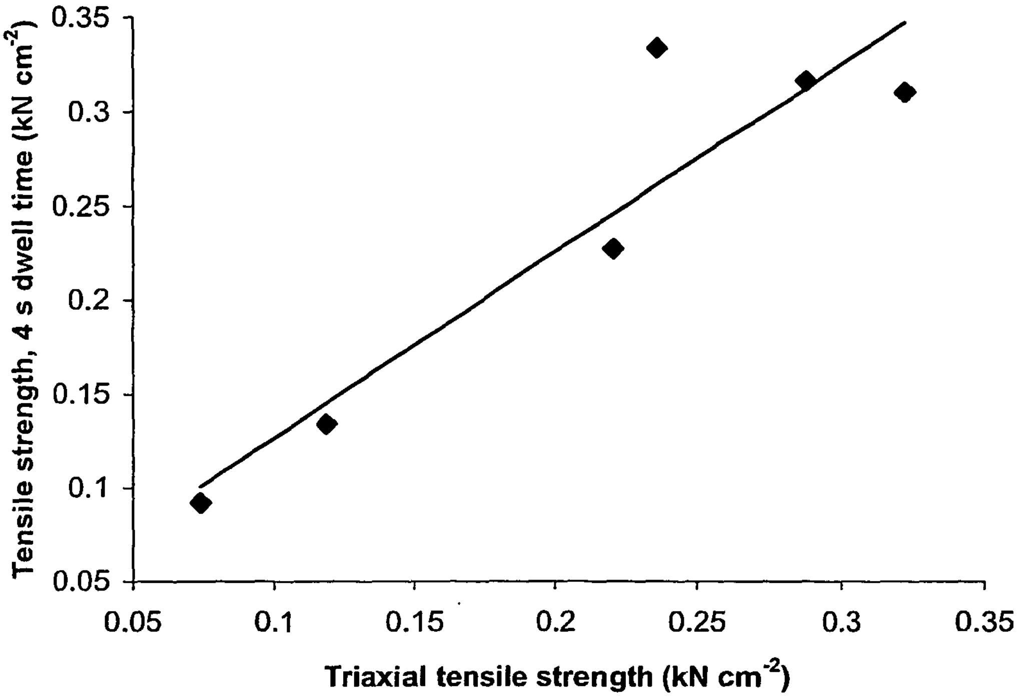
0.35
0.3
0.25
0.2
0.15
0.1
0.05
Tensile strength, 4 s dwell time (kN cm-2)
0.05
0.1
0.15
0.2
0.25
0.3
0.35
Triaxial tensile strength (kN cm-2)

Fig. 1

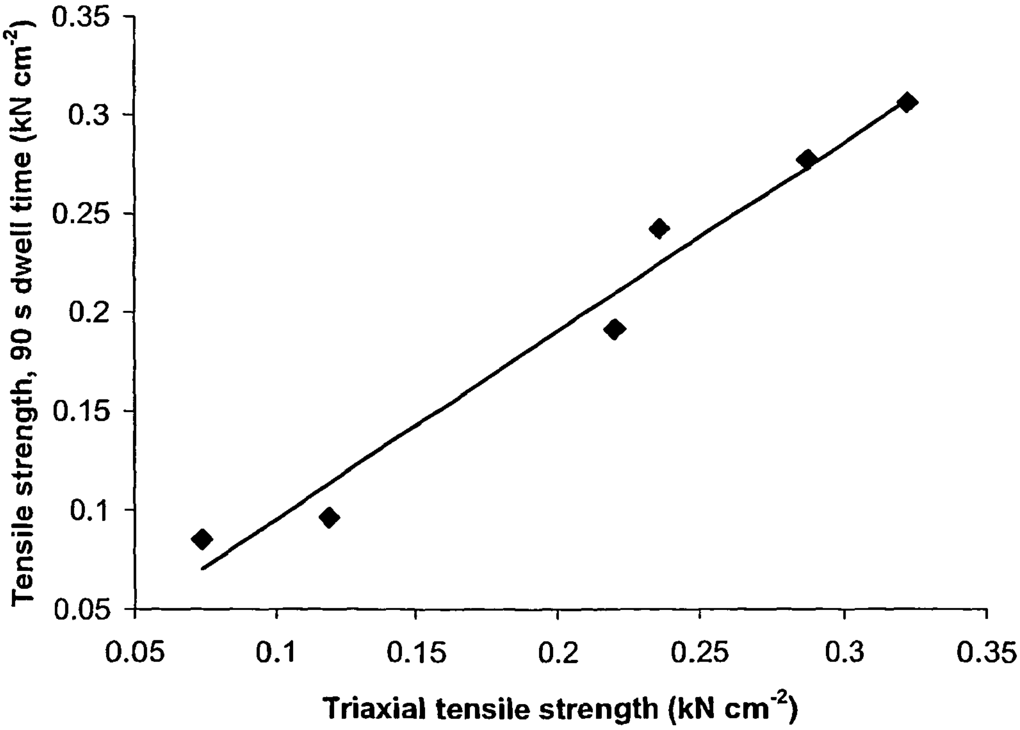
0.35
0.3
0.25
0.2
0.15
0.1
0.05
Tensile strength, 90 s dwell time (kN cm-2)
0.05
0.1
0.15
0.2
0.25
0.3
0.35
Triaxial tensile strength (kN cm-2)

Fig. 2

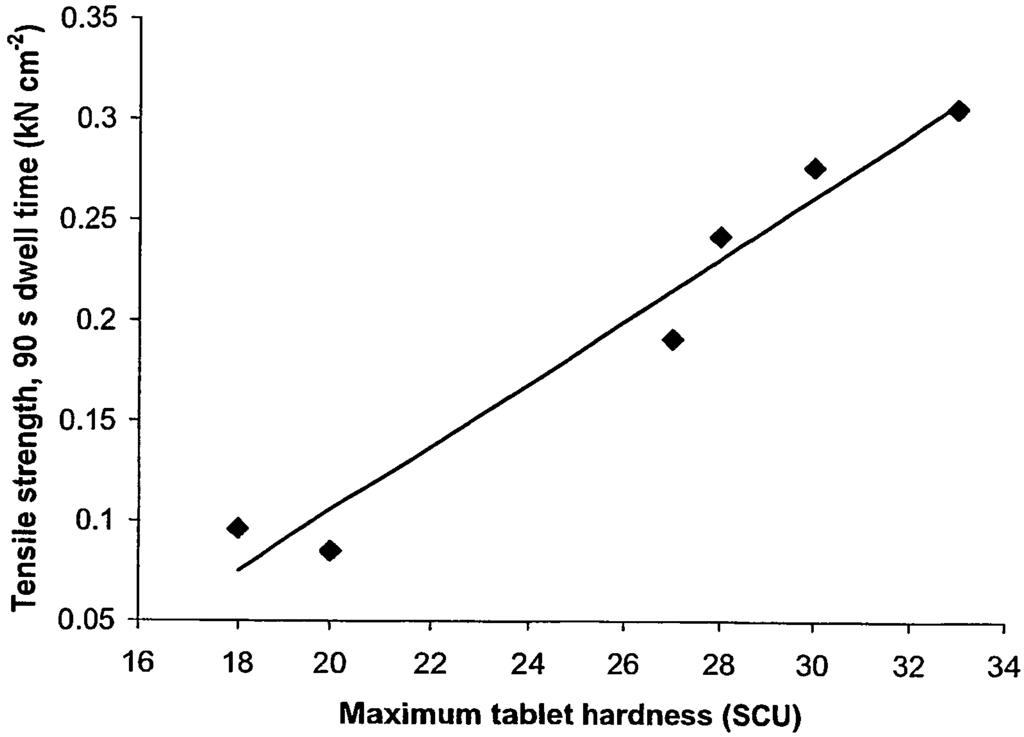

Tensile strength, 90 s dwell time (kN cm-2)
0.35
0.3
0.25
0.2
0.15
0.1
0.05
16
18
20
22
24
26
28
30
32
34
Maximum tablet hardness (SCU)


Fig. 3

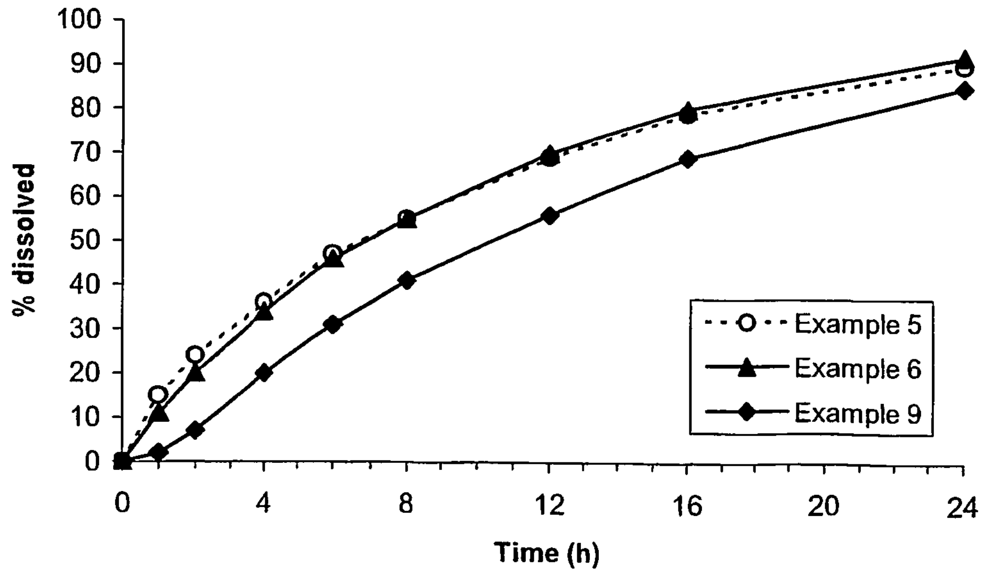

% dissolved
100
90
80
70
60
50
40
30
20
10
0
0
4
8
12
16
20
24
Time (h)
Example 5
Example 6
Example 9


Fig. 4

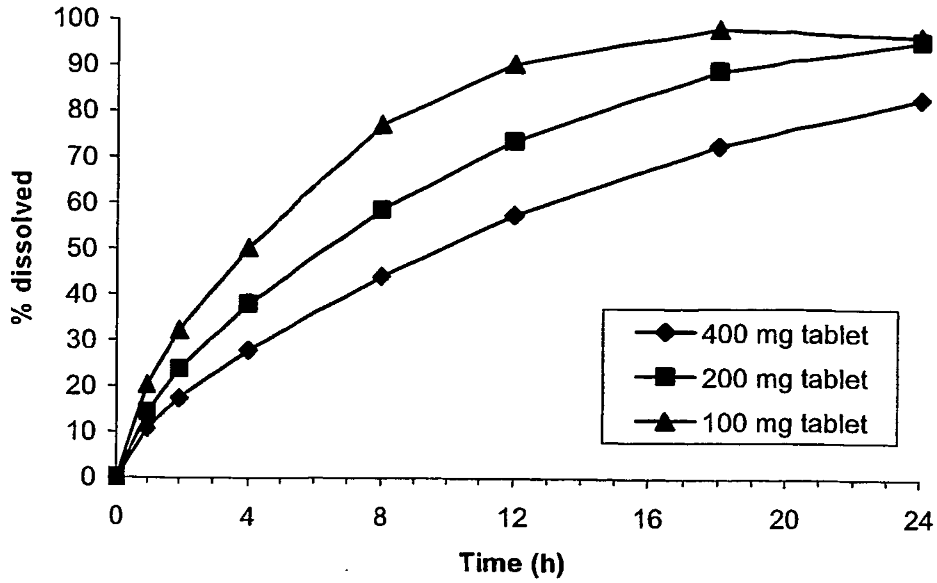
100
90
80
70
60
50
40
30
20
10
0
% dissolved
0
4
8
12
16
20
24
Time (h)
400 mg tablet
200 mg tablet
100 mg tablet

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6197339 B
- US 5458887 A
- CC 5656296
- EP 0933079 A


### Non-patent literature cited in the description


- Physicians' Desk Reference. 2003, 2768-2772
- British National Formulary. 2001, 196
- United States Pharmacopeia. 2000
- **Hiestand ; Smith.** *Powder Technology,* 1984, vol. 38, 145-159
- **Hiestand ; Smith.** *International Journal of Pharmaceutics,* 1991, vol. 67, 231-246
- **Forbes ; Rogers.** *Progress in Neurology and Psychiatry,* 2003, vol. 7 (2), 10-14